# EUROPÄISCHE PATENTSCHRIFT

(11) **EP 0 966 293 B1**
(45) Veröffentlichungstag und Bekanntmachung des Hinweises auf die Patenterteilung: **04.02.2004**
(21) Anmeldenummer: 98954056.2
(22) Anmeldetag: 12.11.1998
(51) Int. Cl.: A61K 35/14, A61K 38/18, A61P 17/02

(54) **ARZNEIMITTEL ZUR FÖRDERUNG DER WUNDHEILUNG ENTHALTEND THROMBOZYTEN**
MEDICINE FOR PROMOTING CICATRIZATION AND CONTAINING THROMBOCYTES
MEDICAMENT FAVORISANT LA CICATRISATION ET CONTENANT DES THROMBOCYTES

(30) Priorität: 12.11.1997 AT 191697
(43) Veröffentlichungstag der Anmeldung: 29.12.1999
(73) Patentinhaber: Bio-Products & Bio-Engineering Aktiengesellschaft, 1010 Wien (AT)
(72) Erfinder: BRAUN, Friedrich, A-1130 Wien (AT); SPÄNGLER, Hans-Peter, A-1180 Wien (AT); EIBL, Johann, A-1180 Wien (AT)
(74) Vertreter: Schwarz, Albin, Dr.
(86) Internationale Anmeldenummer: PCT/AT1998/000278
(87) Internationale Veröffentlichungsnummer: WO 1999/024044

(56) Entgegenhaltungen:
- WO-A-91/17655
- WO-A-97/34614
- DATABASE WPI Section Ch, Derwent Publications Ltd., London, GB; Class B04, AN 73-31168U XP002092218 & SU 353 724 A (ARLOZOROV ZG)
- VALERI C R ET AL: "A SIMPLE METHOD FOR FREEZING HUMAN PLATELETS USING 6% DIMETHYLSULFOXIDE AND STORAGE AT - 80 C" BLOOD, Bd. 43, Nr. 1, 1. Januar 1974, Seiten 131-136, XP000563674

## Beschreibung

Die vorliegende Erfindung betrifft ein Arzneimittel zur lokalen Anwendung für die Förderung der Wundheilung.

Es ist bekannt, daß die Wundheilung in mehreren zeitlich aufeinanderfolgenden Stadien erfolgt.

Im Stadium I wird das Blutplasmaeiweiß Fibrinogen durch Thrombin gefällt, so daß es zur Bildung eines Fibrinclots kommt, der sich in Anwesenheit von Blutgerinnungsfaktor XIII verfestigt. Dieses erste Stadium dient der Blutstillung und Abdichtung der Wundfläche und erfolgt in Minuten.

Im Stadium II wandern in den Fibrinclot Zellen aus dem Wundareal, und zwar Entzündungszellen, Bindegewebszellen und Endothelzellen, ein. Sie bilden Gefäße und als extrazelluläre Matrix Bindegewebe, das vorwiegend aus Kollagen besteht. Dieses als Granulationsgewebe bezeichnete Bindegewebe dient als Unterlage für die Bildung von Epithelgewebe, an der Körperoberfläche ist es die Unterlage für die Epidermis. Stadium II dauert Tage bis Wochen und ist abgeschlossen, wenn das Wundareal durch Epithel, an der Haut durch die Epidermis, verschlossen ist.

Die Wundheilung wird durch das Stadium III, das Wochen bis Monate dauert, abgeschlossen. Im Laufe dieser Phase nehmen die zellulären Elemente ab und das Bindegewebe zu, so daß ein festes und dauerhaftes Narbengewebe entsteht. (Bennett N.T., Schultz G.S., Am. J. Surg. 1993, 165: 728-737; Bennett N.T., Schultz G.S., Am. J. Surg. 1993, 166: 74-81).

Die Bildung von Granulationsgewebe im Stadium II des Wundheilungsprozesses wird durch Wachstumsfaktoren bewirkt, welche die Migration und die Teilung von Bindegewebszellen sowie die Neubildung von Gefäßen fördern und auf diese Weise die Wundheilung vorantreiben. Von den bekannten Wachstumsfaktoren sind insbesondere der Platelet derived growth factor (PDGF), der Transforming growth factor β (TGF-β), der Epidermal growth factor (EGF) und der Insulin-like growth factor I (IGF-I) an diesen Vorgängen beteiligt. (Bennett N.T., Schultz G.S., Am. J. Surg. 1993, 165: 728-737; Bennett N.T., Schultz G.S., Am. J. Surg. 1993, 166: 74-81; Bhora F.Y. et al., J. Surg. Res. 1995, 59: 236-244; Lynch S.E. et al., Proc. Natl. Acad. Sci. USA 1987, 84: 640-646; Lynch S.E. et al., J. Clin. Invest. 1989, 84: 7696-7700).

Auch die Neubildung der Epidermis wird durch Wachstumsfaktoren bewirkt. Sie aktivieren die Epidermiszellen (Keratinozyten), die durch die Verletzung aus dem Zellverband der intakten Basalzellschicht herausgelöst wurden, so daß sie spezifische Membranrezeptoren ausbilden, welche die Anhaftung an die Granulationsgewebsunterlage, besonders an Fibrin-Fibronektin ermöglichen, das ein provisorisches Gerüst für die Migration der Keratinozyten darstellt. (Brown G.L. et al., J. Exp. Med. 1986, 163: 1319-1324; Brown G.L. et al., N. Engl. J. Med. 1989,321: 76-79).

Wachstumsfaktoren werden im menschlichen Körper von verschiedenen Geweben bzw. Zellarten synthetisiert und in die umgebende Körperflüssigkeit sezerniert. Im Rahmen der Wundheilung kommt den Thrombozyten, welche die für die Wundheilung wesentlichen Wachstumsfaktoren PDGF, TGF-β, EGF und IGF-I in signifikanten Mengen synthetisieren und in zytoplasmatischen Granula speichern können, eine wichtige regulatorische Rolle zu. (Lynch S.E. et al., Proc. Natl. Acad. Sci. USA 1987; 84: 640-646; Ginsberg M.H. et al., Thromb. Haemostas. 1988, 59: 1-6; Hyner O.R., Thromb. Haemostas. 1991, 66: 40-43).

Zur Freisetzung bzw. Abgabe der gespeicherten Wachstumsfaktoren aus den Thrombozyten müssen diese durch physiologische Stimuli, wie z.B. Kollagen, Thrombin, Trypsin, ADP, Serotonin oder Adrenalin, welche an spezifische Rezeptoren an der äußeren Oberfläche der Thrombozyten-Plasmamembran binden, aktiviert werden. Die Aktivierung führt zu einer Formänderung mit nachfolgender Aggregation der Thrombozyten, worauf diese die gespeicherten Wachstumsfaktoren in die umgebende Körperflüssigkeit sezernieren. Bei den meisten dieser physiologischen Stimuli ist die der Aktivierung folgende Aggregation der Thrombozyten eine Voraussetzung für die Freisetzung der Wachstumsfaktoren. Bei Stimulation mit Thrombin können die Wachstumsfaktoren auch ohne Thrombozytenaggregation freigesetzt werden. (Kaplan K.L. et al., Blood 1979, 53: 604-618; Holmsen H. et al., J. Biol. Chem. 1981, 256: 9393-9396; Philipps D.R., Baughan A.K., J. Biol. Chem. 1983, 258: 10240-10245).

Die zur Aggregation führenden Wechselwirkungen zwischen den aktivierten Thrombozyten und deren Anheftung an Oberflächen werden durch extrazelluläre adhäsive Matrixproteine, wie z.B. Fibrinogen, Fibronektin und Von-Willebrand-Faktor, vermittelt, die an einen Glykoproteinrezeptor an der Außenseite der Plasmamembran der aktivierten Thrombozyten binden. Eine starke Bindung dieser Matrixproteine an den Rezeptor erfolgt nur, wenn die Thrombozyten wie oben beschrieben durch einen geeigneten Stimulus aktiviert worden sind. Diese komplexen Abläufe von Aktivierung und Aggregation der Thrombozyten mit nachfolgender Freisetzung von Wachstumsfaktoren bilden eines der wesentlichen Steuerungselemente im Wundheilungsprozeß. (Ginsberg M.H. et al., Thromb. Haemostas. 1988, 59: 1-6; Hyner O.R., Thromb. Haemostas. 1991, 66: 40-43; Landolfi R. et al., Blood 1991, 78: 377-381; Perschke E.I. et al., Blood 1980, 55: 841-847; Hynes O.R., Cell 1992, 69: 11-25; Perschke E.I., J. Lab. Clin. Med. 1994, 124: 439-446; Savage B.,Ruggeri Z.M., J. Biol. Chem. 1991, 266: 11227-11233; Bennett J.S. et al., J. Biol. Chem. 1982, 257: 8049-8054; Cierniewski C.S. et al., Biochim. Biophys. Acta 1982, 714: 543-548; Philipps D.R., Baughan A.K., J. Biol. Chem. 1983, 258: 10240-10245).

Störungen der Wundheilung, wie sie etwa bei der Zuckerkrankheit, bei venösen oder arteriellen Verschlußkrankheiten vorkommen, aber auch Wundheilungsstörungen anderer Genese, wie etwa Bestrahlung mit radioaktiven Substanzen oder nach Verbrennungen, betreffen insbesondere das Stadium II des Wundheilungsprozesses. Dabei konnte festgestellt werden, daß in diesen Fällen die Wachstumsfaktoren in einem vermindertem Ausmaß vorhanden sind, sodaß kein oder nur minderwertiges Granulationsgewebe gebildet wird. (Dvonch V.M. et al., Surgery 1992, 112: 18-23; Matsuoka J., Grotendorst G.R., Proc. Natl Acad. Sci. USA 1989, 86: 4416-4420).

Um bei Wundheilungsstörungen die Wundheilung zu verbessern, ist es bekannt, Wachstumsfaktoren einzeln oder in Kombination als Reinsubstanz oder in Salbengrundlagen gemischt auf das Wundareal aufzubringen (Knighton D.R. et al., Surg. Gynecol. Obstet. 1990, 170: 56-60; Brown G.L. et al., J. Exp. Med. 1986, 163: 1319-1324; Holmsen H. et al., J. Biol. Chem. 1981, 256: 9393-9396). Die so zugeführten Wachstumsfaktoren werden jedoch rasch inaktiviert bzw. abgebaut und entfalten ihre Wirkung nur über eine kurze Zeitspanne (Minuten) nach der Applikation, wodurch mit diesen Präparationen keine zufriedenstellende Verbesserung der Wundheilung erzielt werden.

Andere bekannte Therapieansätze bestehen darin, das Wundareal mit Kollagenschwämmen bzw. anderen Präparationen zu bedecken, die eine ständige Feuchtigkeit des Wundareals gewährleisten sollen oder Präparationen zu verwenden, welche die oberflächliche Bindegewebsschicht des Wundareals fermentativ abbauen, sodaß neues Bindegewebe vom Wundgrund aus nachwachsen kann (Nielsen P.G. et al., Acta Dermato-Venerologica 1990, Suppl. 152: 1-12; Lippert P., Wolff H., Zent.bl. Chir. 1990, 115: 1175-1180). Alle diese bisher angewendeten Wundverbände bzw. Präparationen oder Arzneimittel führen jedoch zu keinem zufriedenstellenden Erfolg bei der Verbesserung der Wundheilung.

Die vorliegende Erfindung stellt sich daher die Aufgabe, ein Arzneimittel zur Verfügung zu stellen, welches die natürlichen Wundheilungsvorgänge wirksam beschleunigt und die Wundheilung bei Vorliegen von Wundheilungsstörungen, insbesondere auch bei schweren Formen, im Vergleich zu den bisher bekannten Mitteln und Maßnahmen deutlich verbessern kann.

Diese Aufgabe wird erfindungsgemäß dadurch gelöst, daß ein Arzneimittel zur lokalen Anwendung für die Förderung der Wundheilung bereitgestellt wird, welches unlöslichen Teile von Thrombozyten aufweist, wobei diese Wachstumsfaktoren enthalten und diese abgeben können und in lyophilisiertem oder tiefgefrorenem Zustand vorliegen und einem Verfahren zur Virusabreicherung und/oder Virusinaktivierung unterzogen sind.

Unter "unlöslichen Teile von Thrombozyten" sind alle unlöslichen Thrombozytenbestandteile zu verstehen, die entweder durch Filtration, einschließlich Nanofiltration, oder durch Zentrifugation, einschließlich Ultrazentrifugation, von den löslichen Thrombozytenbestandteilen abtrennbar sind.

Im folgenden bezeichnet - wenn nicht anders angegeben - der Begriff "Thrombozyten" auch "unlöslichen Teile von Thrombozyten".

Die Erfindung beruht auf der Erkenntnis, daß die lokale Anwendung von unlösliche Teilen von Thrombozyten, welche Wachstumsfaktoren enthalten und diese abgeben können, die Wundheilungsvorgänge wirksam beschleunigen kann. Die auf das Wundareal aufgebrachten Thrombozytenteilen stellen ein natürliches Reservoir für die zur Förderung der Wundheilungsprozesse benötigten Wachstumsfaktoren dar. Es hat sich gezeigt, daß die Aktivierung der lokal applizierten Thrombozytenteilen durch im Wundareal vorhandene physiologische Stimuli mit nachfolgender Aggregation und Bindung der im Wundareal vorhandenen Matrixproteine dazu führt, daß die in den Thrombozytenteilen gespeicherten Wachstumsfaktoren kontinuierlich über einen längeren Zeitraum (mehrere Tage) in das Wundareal abgegeben werden. Dadurch stehen offenbar höhere Konzentrationen an Wachstumsfaktoren über einen wesentlich längeren Zeitraum als bei direkter Gabe der Wachstumfaktoren im Wundareal zur Verfügung, wodurch das Einwandern von Entzündungszellen, Bindegewebszellen und Endothelzellen gefördert und die Vermehrung dieser Zellen im Stadium II des Wundheilungsprozesses gesteigert wird. Auf diese Weise kommt es zu einer raschen und ausreichenden Bildung von Granulationsgewebe, was wiederum die Ausbildung von Epithelgewebe und den endgültigen Wundverschluß ermöglicht. Der Epithelisierungsvorgang wird außerdem durch die freigesetzten Wachstumsfaktoren, welche die Einwanderung und Vermehrung von Epithelzellen fördern, zusätzlich beschleunigt.

Um die Haltbarkeit des Arzneimittels über einen längeren Zeitraum zu gewährleisten, liegen die Thrombozytenteilen im erfindungsgemäßen Arzneimittel vorzugsweise in lyophilisiertem oder tiefgefrorenem Zustand vor. Zur Minimierung des Risikos von Virusinfektionen werden die Thrombozyten vorteilhaft einem Verfahren zur Virusabreicherung und/oder Virusinaktivierung unterzogen, wobei ein physikalisches oder chemisches Verfahren oder ein Kombinationsverfahren angewendet werden kann.

Zur Bereitstellung einer höheren Konzentration an Wachstumsfaktoren, insbesondere in der Behandlung von Wundheilungsstörungen, ist es bevorzugt, daß der Gehalt an unlösliche Teilen von Thrombozyten des erfindungsgemäßen Arzneimittels derart ist, daß er nach Rekonstitution des Lyophilisates bzw. nach Auftauen mindestens 10⁴, vorzugsweise mindestens 10⁵, Thrombozyten pro µl entspricht.

Um eine besonders ausgeprägte Initialwirkung des erfindungsgemäßen Arzneimittels unmittelbar nach Applikation zu erzielen, kann es, insbesondere bei schweren Wundheilungsstörungen, sinnvoll sein, daß das Arzneimittel weitere Wachstumsfaktoren aufweist, welche nicht aus den im Arzneimittel enthaltenen Thrombozyten stammen. Die weiteren Wachstumsfaktoren können vom selben Typ sein wie die von den Thrombozyten des erfindungsgemäßen Arzneimittels gespeicherten und abgegebenen Wachstumsfaktoren oder einem unterschiedlichen Typ angehören. Die Wachstumsfaktoren können mit den Thrombozyten im gleichen Behältnis vorliegen oder als Lösung oder Lyophilisat in einem separaten Behältnis enthalten sein.

Es hat sich gezeigt, daß es insbesondere bei ausgeprägten Fällen von Wundheilungsstörungen vorteilhaft ist, wenn das Arzneimittel Biomaterialien aufweist. Unter Biomaterialien im Sinne der Erfindung sind alle Materialien zu verstehen, welche gewebeverträglich und resorbierbar sind und im Zusammenwirken mit den im Arzneimittel enthaltenen Thrombozyten oder Wachstumsfaktoren oder unabhängig davon die Förderung der Wundheilung unterstützen. So können beispielsweise Substanzen, welche als Stimuli Thrombozyten aktivieren, und/oder Materialien, welche die Aggregation von Thrombozyten vermitteln, als Biomaterialien im erfindungsgemäßen Arzneimittel enthalten sein. Auf diese Weise wird die Wirkung der natürlichen, im Wundareal vorhandenen aktivierenden und aggregationsvermittelnden Substanzen verstärkt, wodurch die Freisetzung von Wachstumsfaktoren erhöht und die Wundheilung noch stärker gefördert werden.

Zur Minimierung des Risikos von Virusinfektionen werden die Biomaterialien vorzugsweise einem Verfahren zur Virusabreicherung und/oder Virusinaktivierung unterzogen, wobei ein physikalisches oder chemisches Verfahren oder ein Kombinationsverfahren angewendet werden kann. Die Biomaterialien können einem solchen Verfahren einzeln oder im Gemisch mit anderen Bestandteilen des Arzneimittels (z.B. Thrombozyten) unterworfen werden.

Um die Haltbarkeit des Arzneimittels über einen längeren Zeitraum zu gewährleisten, liegen die Biomaterialien im erfindungsgemäßen Arzneimittel vorteilhaft in lyophilisiertem oder tiefgefrorenem Zustand vor. Dabei können die Biomaterialien mit den Thrombozyten und/oder Wachstumsfaktoren in gemeinsamen Behältnissen vorliegen oder in getrennten Behältnissen enthalten sein und das Tieffrieren bzw Lyophilisieren der Biomaterialien an diesen einzeln oder im Gemisch mit anderen Bestandteilen des Arzneimittels vorgenommen werden.

Es ist bekannt, daß die Aktivierung und Aggregation von Thrombozyten und damit die Freisetzung von in den Thrombozyten gespeicherten Wachstumsfaktoren durch die Anlagerung von Matrixproteinen ermöglicht wird. Außerdem können solche Proteine vernetzte Strukturen ausbilden, an denen die Thrombozyten anhaften und sich mit dem Wundareal fest verbinden, wobei diese Strukturen die Diffusion der Wachstumsfaktoren zum Wundareal und das Einwandern der Zellen aus dem Wundareal fördern. Demgemäß ist eine bevorzugte Ausführungsform des erfindungsgemäßen Arzneimittels dadurch gekennzeichnet, daß als Biomaterialien Gewebeklebstoff und/oder Kollagen vorgesehen sind. Unter Gewebeklebstoffim Sinne der Erfindung werden Biomaterialien verstanden, die ganz oder teilweise aus vernetzbaren Proteinen bestehen, welche zur Gewebeklebung geeignet sind.

Fibrinogen ist eine besonders wirksame Substanz zur Auslösung der Aggregation aktivierter Thrombozyten, während Thrombin eine der wirksamsten Substanzen für die Aktivierung von Thrombozyten darstellt. Es ist daher vorteilhaft für die Steigerung der Freisetzung von Wachstumsfaktoren und die Verbesserung der Wundheilung, daß der Gewebeklebstoff aus fibrinogenhältigen Proteinen und Thrombin zusammengesetzt ist.

Es hat sich gezeigt, daß humane Zellen, wie Keratinozyten, Epithelzellen, embryonale und fetale Zellen, sowie Zellbestandteile, wie Liposomen, die durch Thrombozyten geförderte Wundheilung und Zellvermehrung noch zusätzlich beschleunigen können. Es ist daher bevorzugt, daß das Arzneimittel zusätzlich Epithelzellen und/oder Keratinozyten und/oder embryonale und/oder fetale Zellen und/oder Liposomen aufweist. Die Zellen bzw. Liposomen können als flüssige oder tiefgefrorene Suspension oder als Lyophilisat in getrennten Behältnissen oder eine oder mehrere der genannten Zellarten oder Liposomen entweder ohne oder mit einem der anderen Bestandteile des Arzneimittels in gemeinsamen Behältnissen vorliegen.

Zur Minimierung des Risikos von Virusinfektionen können die Zellen bzw. Liposomen einem Verfahren zur Virusabreicherung und/oder Virusinaktivierung unterzogen sein, wobei ein physikalisches oder chemisches Verfahren oder ein Kombinationsverfahren angewendet werden kann. Die Zellen bzw. Liposomen können einem solchen Verfahren einzeln oder im Gemisch mit anderen Bestandteilen des Arzneimittels unterworfen werden.

Die Erfindung betrifft auch die Verwendung von unlösliche Teilen von Thrombozyten, welche Wachstumsfaktoren enthalten, zur Herstellung eines Arzneimittels zur lokalen Anwendung für die Förderung der Wundheilung.

Bevorzugte Ausführungsformen der Erfindung werden im folgenden anhand von Beispielen näher erläutert.

### Beispiel 1: Herstellung eines erfindungsgemäßen Arzneimittels

Humanes Thrombozytenkonzentrat oder Konzentrat aus Thrombozytenbestandteilen wird durch 3% Natriumcitrat antikoaguliert und zentrifugiert (1000 g/ 20 min), um Plasma und andere Zellbestandteile zu entfernen. Der thrombozytenreiche Überstand bzw. der Überstand an Thrombozytenbestandteilen wird in RPMI-Medium suspendiert und dreimal in RPMI-Medium (1000 g/ 20 min) gewaschen. Die gewaschenen Thrombozyten bzw. die gewaschenen Thrombozytenbestandteile werden in einem RPMI-Medium suspendiert und auf eine Konzentration von mindestens 6x10⁵ Thrombozyten bzw Thrombozytenbestandteilen pro µl eingestellt. Die Thrombozytensuspension wird sodann einem Virusinaktivierungsverfahren gemäß Beispiel 3 unterzogen und anschließend entsprechend den unten beschriebenen Verfahren tiefgefroren oder lyophilisiert, wodurch ein erfindungsgemäßes Arzneimittel erhalten wird.

Tieffrierung: Je 1 ml der Thrombozytensuspension wird bei -80°C innerhalb von 30-40 Minuten schock-tiefgefroren und tiefgefroren gelagert. Vor Verwendung wird das Thrombozytenkonzentrat bei Zimmertemperatur aufgetaut.

Lyophilisierung: Je 1 ml der Thrombozytensuspension wird bei -80° für mindestens 24 Stunden tiefgefroren und anschließend bei -20°C bis -40°C über 20 bis 24 Stunden unter Vakuum gefriergetrocknet. Die gefriergetrockneten Thrombozyten werden zwischen -20°C und -80°C gelagert und vor Verwendung mit 1 ml RPMI-Medium rehydriert.

### Beispiel 2: Herstellung eines erfindungsgemäßen Arzneimittels, das Biomaterialien aufweist

Der gemäß Beispiel 1 hergestellten virusinaktivierten Thrombozytensuspension wird eine Lösung von vernetzbarem humanen Eiweiß (entweder Fibrinogen, Fibronektin, Blutgerinnungsfaktor XIII oder Kollagen), das einem oder mehreren Verfahren zur Virusinaktivierung gemäß Beispiel 4 unterzogen worden sein kann, zugesetzt und zwar jeder Eiweißkörper einzeln oder miteinander in Kombination, wobei die Konzentration der vernetzbaren Eiweißkörper in der zugesetzten Lösung vorzugsweise 70-90 mg/ml betragen soll. Das Mischungsverhältnis von Thrombozytensuspension zur Lösung von vernetzbarem humanen Eiweiß soll vorzugsweise 1:3 betragen. Die so erhaltene Mischung wird zur Erzielung einer zweckmäßigen Haltbarkeit gemäß den in Beispiel 1 dargestellten Verfahren tiefgefroren oder lyophilisiert.

Anstelle der Durchführung der Virusinaktivierung an den Einzelkomponenten (Thrombozyten bzw. Biomaterialien) ist es auch möglich, die Virusinaktivierung an dem Gemisch von Thrombozytensuspension und Proteinlösung gemäß dem Verfahren von Beispiel 3 vorzunehmen.

### Beispiel 3: Virusinaktivierung der Thrombozytensuspension (Photodynamische Virusinaktivierung)

Zu 50 ml der gemäß Beispiel 1 hergestellten Thrombozytensuspension wird 8-Methoxypsoralen (gelöst in Dimethylsulfoxid [DMSO]) bis zu einer Endkonzentration von 300 µg/ml (Endkonzentration von DMSO 0,3 %) zugesetzt und bei 22-27°C unter einer Atmosphäre von 5% CO₂ und 95% N₂ und einem Druck von 2 psi 6 Stunden lang mit ultraviolettem Licht von unten und oben bestrahlt, sodaß die gesamte Lichtintensität 3,5 bis 4,8 mW/cm² beträgt (Lin L. et al., Blood 1989, 74: 517-525).

Nach durchgeführter Photoinaktivierung werden die so erhaltenen Thrombozytensuspensionen auf ihre funktionelle Kapazität untersucht. Die funktionelle Kapazität wird durch Messung des [³H]-Thymidineinbaus in einer Fibroblastenzellkultur bestimmt.

### Beispiel 4: Virusinaktivierung der Biomaterialien (Chemische Virusinaktivierung)

Biomaterialien, die der gemäß Beispiel 1 hergestellten Thrombozytensuspension zugesetzt werden, werden durch die Solvent-Detergent-Methode virusinaktiviert. Dazu wird einer Suspension der Biomaterialien bei 30°C 1% (Gew./Gew.) Tri(n-butyl)phosphat und 1% (Gew./Gew.) Triton X-100 zugesetzt und das Gemisch 4 Stunden unter Schütteln belassen. Danach wird unter Zusatz von 5 % (Vol./Vol.) Sojabohnenöl die Solvent-Detergent-Mischung aus der Suspension der Biomaterialien an einer C18-Säule (Waters Millipore) durch Chromatographie entfernt (Horowitz B. et al., Blood 1992, 79: 826-831; Piet M.P.J. et al., Transfusion 1990, 30: 591-598; Piquet Y. et al., Vox sang. 1992, 63: 251-256).

Die mit der oben beschriebenen chemischen Virusinaktivierungsmethode behandelten Biomaterialien können nachfolgend zusätzlich noch einer photodynamischen Virusinaktivierung unterzogen werden.

### Beispiel 5: Nachweis der Förderung der Bindegewebsvermehrung durch das erfindungsgemäße Arzneimittel

Der Test wurde an einer Fibroblastenzellkultur durchgeführt. Auf einer Zellkulturplatte wurde das gemäß Beispiel 2 hergestellte erfindungsgemäße Arzneimittel in einer Menge von 200 µl pro cm² aufgetragen und durch 50 µl einer Thrombinlösung (3,2 IU Thrombin pro ml physiologischer Kochsalzlösung) aktiviert. Auf die aufgetragene Suspension wurden humane Fibroblasten, die aus der 4. bis 10. Passage einer primären Kultur stammten, in einer Dichte von 4 x 10⁴ Zellen pro cm² gesetzt und in einem Zellkulturmedium (RPMI) kultiviert (Kultur 1). Am dritten, fünften und siebenten Tag der Kultivierung wurde die Zell-Mitoserate durch Messung der DNA-Synthese über den Einbau von [³H]-Thymidin gemessen. Die Zell-Mitoserate von Kultur 1 wurde mit der Zell-Mitoserate einer anderen Fibroblastenkultur (Kultur 2) verglichen, die in einem RPMI-Nährmedium, dem 10 Vol% Kalbserum beigesetzt worden waren, ohne Zusatz des erfindungsgemäßen Arzneimittels erfolgte.

Ergebnisse: Kultur 1 zeigte am Tag 3 der Kultivierung einen 7-fach höheren [³H]-Thymidineinbau (196645 ± 56864 cpm/ml) als Kultur 2. An den Tagen 5 (152749 ± 93951 cpm/ml) und 7 (77045 ± 27974 cpm/ml) war der [³H]-Thymidineinbau bei Kultur 1 immer noch 5- bis 10-fach höher als bei Kultur 2. Diese Unterschiede zwischen Kultur 1 und Kultur 2 sind statistisch hochsignifikant (p<0,01) und demonstrieren die Fähigkeit des erfindungsgemäßen Arzneimittels, die Bindegewebsvermehrung zu fördern und diese Aktivität über einen längeren Zeitraum (zumindest 7 Tage) aufrecht zu erhalten.

### Beispiel 6: Nachweis, daß die Bindung von Matrixproteinen an den Thrombozytenoberflächen dazu führt, daß die in den Thrombozyten gespeicherten Wachstumsfaktoren kontinuierlich abgegeben werden.

Der Test wurde an einer Fibroblastenkultur (gemäß Beispiel 5) durchgeführt. In Kultur 1 war - wie in Beispiel 5 - das erfindungsgemäße Arzneimittel zugesetzt. In Kultur 2 wurden die Thrombozyten mit spezifischen Antikörpern gegen die oberflächlichen Bindungsstellen für Matrixproteine behandelt, so daß die Matrixproteine nicht an die Thrombozytenoberflächen binden konnten. Am dritten Tag der Kultivierung wurde die Zell-Mitoserate durch Messung der DNA-Synthese über den Einbau von [³H]-Thymidin gemessen.

Ergebnisse: Während Kultur 1 eine Thymidineinbaurate ähnlich wie in Beispiel 5 aufwies, konnte in Kultur 2 kein Thymidineinbau gemessen werden. Dieser Unterschied beweist, daß für die Freisetzung der in den Thrombozyten gespeicherten Wachstumsfaktoren die Bindung von Matrixproteinen an der Thrombozytenoberfläche notwendig ist.

### Beispiel 7: Nachweis der Förderung der Wundheilung durch das erfindungsgemäße Arzneimittel

Die klinische Wirksamkeit des erfindungsgemäßen Arzneimittels wurde an 6 Patienten mit chronischen, nicht heilenden cutanen Ulcera der unteren Extremitäten, die bereits länger als ein halbes Jahr mit chirurgischer oder konservativ-lokaler Therapie erfolglos behandelt worden waren, geprüft. Die Ulcera wurden nach einem von Knighton D.R. et al., Ann. Surg. 1986, 204: 322-330, angegebenen Wundscore klassifiziert. Der Wundscore beinhaltet allgemeine Parameter, anatomische Bedingungen und Meßgrößen des Ulcus. Je höher die Punktezahl, desto schlechter die Voraussetzungen für die Heilung; es können 97 Punkte als Maximum (=schlechteste Ausgangssituation) erreicht werden.

### Behandlungsplan:

Die Ulcera wurden gereinigt, nekrotisches Gewebe entfernt und mit Thrombinlösung (3,2 IU bovines Thrombin/ml RPMI-Medium) benetzt. Anschließend wurde der Defekt mit dem gemäß Beispiel 2 hergestellten, aufgetauten erfindungsgemäßen Arzneimittel aufgefüllt und darauf zur Aktivierung der Thrombozyten die oben genannte Thrombinlösung in einem Volumsverhältnis Arzneimittelsuspension zu Thrombinlösung von 3:1 aufgetragen. Die so versorgten Ulcera wurden mit einem nicht haftenden Wundverband (Metallfolie) bedeckt. Bis zur Abheilung wurden die Ulcera in oben angegebener Art zweimal pro Woche behandelt. Der Heilungsfortschritt wurde fotografisch und histologisch (Feinnadelbiopsien in der 2. und 5. Behandlungswoche) dokumentiert.

### Ergebnisse:

Die Patientendaten, ursächliche Gefäß- und Stoffwechselerkrankungen sowie die Auswertung der Wundscores am Behandlungsbeginn sind in Tabelle 1 zusammengefaßt.

**Tabelle 1**

| | | | Gefäßerkrankung | | Stoffwechselerkrankung | |
|---|---|---|---|---|---|---|
| Patient | Geschlecht | Alter | arteriell | venös | | Wundscore |
| 1 | männlich | 67 | + | + | Diabetes | 51 |
| 2 | männlich | 72 | + | - | - | 65 |
| 3 | männlich | 69 | + | - | Diabetes | 33 |
| 4 | männlich | 63 | + | - | Diabetes | 49 |
| 5 | männlich | 78 | + | + | Diabetes | 63 |
| 6 | weiblich | 74 | - | + | - | 65^{a}/63^{b} |

| | | | | | | |
|---|---|---|---|---|---|---|
| ^{a,b}) zwei Ulcera an einem Bein: ^{a}) proximales, ^{b}) distales Ulcus | | | | | | |

Der zeitliche Ablauf der Wundheilung (angegeben in Wochen nach Behandlungsbeginn) ist in Tabelle 2 dargestellt.

**Tabelle 2**

| Patient | Beginn der Granulationsgewebsbildung | Beginn der Epithelisierung | Abschluß der Epithelisierung |
|---|---|---|---|
| 1 | 1. Woche | 3. Woche | 8. Woche |
| 2 | 1. Woche | 3. Woche | 9. Woche |
| 3 | 3. Woche | 8. Woche | 12. Woche |
| 4 | 1. Woche | 4. Woche | 10. Woche |
| 5 | 1. Woche | kein | kein |
| 6 | ^{a,b}1. Woche | ^{a}6./^{b}3. Woche | ^{a}12./^{b}9. Woche |

| | | | |
|---|---|---|---|
| ^{a,b}) zwei Ulcera an einem Bein: ^{a}) proximales, ^{b}) distales Ulcus | | | |

Mit Ausnahme von Patient 3 bildete sich bei allen Patienten vom Ulcusboden her bereits in der ersten Behandlungswoche ein gut durchblutetes Granulationsgewebe aus, das nach weiteren Behandlungen mit dem erfindungsgemäßen Arzneimittel bis etwa zwei Wochen nach Therapiebeginn zunahm und das Ulcus ausfüllte. Auffallend war, daß sich bei allen Patienten bereits nach den ersten Behandlungen die Umgebung des Ulcus beruhigte, das Erythem und das Ödem der umliegenden Haut verschwanden und auch der Ulcusrand nicht mehr ödematös und mißfarben war. Histologisch zeigte sich bei allen Biopsien in der zweiten Behandlungswoche zellreiches, vorwiegend aus Fibroblasten und Fibrozyten bestehendes Granulationsgewebe mit reichlicher Gefäßneubildung und kollagener Faserbildung und mit nur oberflächlich geringer Infiltration von Entzündungszellen und Gewebsnekrosen. Eine Epithelisierung der Hautdefekte ging nach der dritten Behandlungswoche von den Wundrändern aus und konnte dann auch histologisch bei den zweiten Biopsien in der fünften Behandlungswoche nachgewiesen werden. Im weiteren Verlauf der Behandlung nahm das Ausmaß der Ulcera einerseits durch die Epithelisierung, aber auch durch eine narbige Schrumpfung ab. Sie heilten mit Ausnahme bei Patient 5 spätestens in der 12. Behandlungswoche narbig ab.

Die oben angeführten Ergebnisse zeigen, daß die lokale Anwendung des erfindungsgemäßen Arzneimittels bei Patienten, die mit konservativer Therapie mindestens ein halbes Jahr lang erfolglos behandelt worden waren und daher extrem schlechte Voraussetzungen für eine Wundheilung aufweisen, die Wundheilung fördern und auf diese Weise chronisch nichtheilende, cutane Ulcera zur Ausheilung bringen kann.

## Patentansprüche

1. Arzneimittel zur lokalen Anwendung für die Förderung der Wundheilung, welches unlöslichen Teile von Thrombozyten aufweist, wobei diese
- Wachstumsfaktoren enthalten und diese abgeben können,
- in lyophilisiertem oder tiefgefrorenem Zustand vorliegen, und
- einem Verfahren zur Virusabreicherung und/oder Virusinaktivierung unterzogen sind.

2. Arzneimittel nach Anspruch 1, **dadurch gekennzeichnet, daß** der Gehalt an unlöslichen Teilen von Thrombozyten derart ist, daß er nach Rekonstitution des Lyophilisates bzw. nach Auftauen mindestens 10⁴, vorzugsweise mindestens 10⁵, Thrombozyten pro ml entspricht.

3. Arzneimittel nach Anspruch 1 oder 2, **dadurch gekennzeichnet, daß** es weitere Wachstumsfaktoren aufweist.

4. Arzneimittel nach einem der Ansprüche 1 bis 3, **dadurch gekennzeichnet, daß** es Biomaterialien aufweist.

5. Arzneimittel nach Anspruch 4, **dadurch gekennzeichnet, daß** die Biomaterialien einem Verfahren zur Virusabreicherung und/oder Virusinaktivierung unterzogen sind.

6. Arzneimittel nach Anspruch 4 oder 5, **dadurch gekennzeichnet, daß** die Biomaterialien in lyophilisiertem oder tiefgefrorenem Zustand vorliegen.

7. Arzneimittel nach einem der Ansprüche 4 bis 6, **dadurch gekennzeichnet, daß** als Biomaterialien Gewebeklebstoff und/oder Kollagen vorgesehen sind.

8. Arzneimittel nach Anspruch 7, **dadurch gekennzeichnet, daß** der Gewebeklebstoff aus fibrinogenhältigen Proteinen.und Thrombin zusammengesetzt ist.

9. Arzneimittel nach einem der Ansprüche 4 bis 8, **dadurch gekennzeichnet, daß** es zusätzlich Epithelzellen und/oder Keratinozyten und/oder embryonale und/oder fetale Zellen und/oder Liposomen aufweist.

10. Verwendung von unlöslichen Teilen von Thrombozyten, welche Wachstumsfaktoren enthalten und diese abgeben können, zur Herstellung eines Arzneimittels zur lokalen Anwendung für die Förderung der Wundheilung.

## Claims

1. A drug composition to be applied topically for the promotion of wound healing, comprising insoluble parts of thrombocytes, wherein those parts
- contain growth factors and are able to release them,
- are provided in freeze-dried or deep-frozen state, and
- are subjected to a procedure for virus depletion and/or virus inactivation.

2. A drug composition according to claim 1, **characterized in that** the content of insoluble parts of thrombocytes is such that, after the reconstitution of the lyophilisate or after thawing, respectively, it corresponds to at least 10⁴, preferably at least 10⁵, thrombocytes per ml.

3. A drug composition according to claim 1 or 2, **characterized in that** it comprises further growth factors.

4. A drug composition according to any of claims 1 to 3, **characterized in that** it comprises biomaterials.

5. A drug composition according to claim 4, **characterized in that** the biomaterials are subjected to a procedure for virus depletion and/or virus inactivation.

6. A drug composition according to claim 4 or 5, **characterized in that** the biomaterials are provided in freeze-dried or deep-frozen state.

7. A drug composition according to any of claims 4 to 6, **characterized in that**, as. biomaterials, a tissue adhesive and/or collagen is/are provided.

8. A drug composition according to claim 7, **characterized in that** the tissue adhesive is composed of fibrinogen-containing proteins and thrombin.

9. A drug composition according to any of claims 4 to 8, **characterized in that**, in addition, it comprises epithelial cells and/or keratinocytes and/or embryonic and/or fetal cells and/or liposomes.

10. The use of insoluble parts of thrombocytes, which contain growth factors and are able to release them, for the preparation of a drug composition to be applied topically for the promotion of wound healing.

## Revendications

1. Médicament destiné à une application locale pour favoriser la cicatrisation, et comportant des parties insolubles de thrombocytes, qui
- contiennent des facteurs de croissance et peuvent libérer ceux-ci,
- sont présents à l'état lyophilisé ou surgelé, et
- qui sont soumis à un procédé pour un appauvrissement viral et/ou une inactivation virale.

2. Médicament selon la revendication 1, **caractérisé en ce que** la teneur en parties insolubles de thrombocytes est telle que, après reconstitution du lyophilisat ou bien après décongélation, elle corresponde à au moins 10⁴ et, de préférence, à au moins 10⁵ thrombocytes par ml.

3. Médicament selon la revendication 1 ou 2, **caractérisé en ce qu'**il comporte des facteurs supplémentaires de croissance.

4. Médicament selon l'une des revendications 1 à 3, **caractérisé en ce qu'**il comporte des biomatériaux.

5. Médicament selon la revendication 4, **caractérisé en ce que** les biomatériaux sont soumis à un procédé pour un appauvrissement viral et/ou une inactivation virale.

6. Médicament selon la revendication 4 ou 5, **caractérisé en ce que** les biomatériaux sont présents à l'état lyophilisé ou surgelé.

7. Médicament selon l'une des revendications 4 à 6, **caractérisé en ce qu'**il est prévu, en tant que biomatériaux, une substance adhésive pour tissus et/ou un collagène.

8. Médicament selon la revendication 7, **caractérisé en ce que** la substance adhésive pour tissus est composée de protéines contenant des fibrinogènes, et de thrombine.

9. Médicament selon l'une des revendications 4 à 8, **caractérisé en ce qu'**il comporte, en outre, des cellules épithéliales et/ou des kératinocytes et/ou des cellules embryonnaires et/ou foetales et/ou des liposomes.

10. Utilisation de parties insolubles de thrombocytes, qui contiennent des facteurs de croissance et peuvent libérer ceux-ci, pour la fabrication d'un médicament destiné à une application locale pour favoriser la cicatrisation.
